# FASCICULE DE BREVET EUROPEEN

(11) **EP 4 078 464 B1**
(45) Date de publication et mention de la délivrance du brevet: **27.12.2023**
(21) Numéro de dépôt: 20848712.4
(22) Date de dépôt: 17.12.2020
(51) Int. Cl.: G06N 3/08, G16H 30/40, A61B 34/10, A61B 34/20, A61B 90/00

(54) **MÉTHODE DE PLANIFICATION AUTOMATIQUE D'UNE TRAJECTOIRE POUR UNE INTERVENTION MÉDICALE**
VERFAHREN ZUM AUTOMATISCHEN PLANEN EINER TRAJEKTORIE FÜR EINEN MEDIZINISCHEN EINGRIFF
METHOD FOR AUTOMATICALLY PLANNING A TRAJECTORY FOR A MEDICAL INTERVENTION

(30) Priorité: 18.12.2019 FR 1914780
(43) Date de publication de la demande: 26.10.2022
(73) Titulaire: Quantum Surgical, 34000 Montpellier (FR)
(72) Inventeur: NAHUM, Bertin, 34170 CASTELNAU-LE-LEZ (FR); BADANO, Fernand, 69006 LYON (FR); BLONDEL, Lucien, 34070 MONTPELLIER (FR)
(74) Mandataire: Ipside
(86) Numéro de dépôt international: PCT/FR2020/052513
(87) Numéro de publication internationale: WO 2021/123651

(56) Documents cités:
- WO-A1-2018/223925
- WO-A2-2012/092511
- US-A1- 2017 148 213
- US-A1- 2019 262 084
- US-A1- 2019 307 516

## Description

### DOMAINE TECHNIQUE DE L'INVENTION

Le domaine de l'invention est celui de l'assistance à la planification d'une intervention médicale.

Plus précisément, l'invention concerne un procédé de planification automatique d'une trajectoire d'un instrument médical à effectuer au cours d'une intervention médicale et un dispositif de guidage associé.

L'invention trouve notamment des applications dans le cadre d'une intervention médicale au cours de laquelle un instrument médical est inséré dans une anatomie d'intérêt, par exemple pour ablater une tumeur dans un organe, pour réaliser une biopsie, pour effectuer une vertébroplastie ou une cimentoplastie, voire pour stimuler une zone anatomique. Une telle intervention peut éventuellement être assistée par un robot médical et/ou par un dispositif de réalité augmentée.

### ÉTAT DE LA TECHNIQUE

Il est connu de l'art antérieur des techniques permettant de préparer une intervention médicale visant à atteindre une zone anatomique cible dans une anatomie d'intérêt d'un patient, telle qu'un poumon, un rein, un foie, un cerveau, un tibia, un genou, une vertèbre, etc.

Traditionnellement, la planification de l'intervention médicale est effectuée manuellement par un opérateur à partir d'une image médicale obtenue par une méthode d'imagerie médicale courante.

Lors de la planification, l'opérateur définit un point cible dans l'anatomie d'intérêt et un point d'entrée sur la peau du patient à proximité de l'anatomie d'intérêt, les deux points définissant une trajectoire rectiligne d'un instrument médical utilisé lors de l'intervention médicale. Un tel instrument peut être par exemple une aiguille, une sonde ou une électrode.

L'opérateur doit être attentif à la trajectoire que va prendre l'instrument médical car elle doit respecter un certain nombre de contraintes nécessaires au bon déroulement de l'intervention médicale. Par exemple, il peut être important que l'instrument médical ne traverse pas d'os ou de vaisseaux sanguins, notamment ceux dont le diamètre est supérieur à trois millimètres, ou ne traverse pas d'organes vitaux.

Afin d'aider l'opérateur dans le choix du point d'entrée en fonction du point cible, il a été développé des techniques de planification dans laquelle un ou plusieurs points d'entrée sont proposés automatiquement à un opérateur en fonction de contraintes préalablement définies, en associant à chaque trajectoire correspondante un score en fonction de critères prédéfinis.

Une telle technique est par exemple décrite dans la demande de brevet américain publié sous le numéro US 2017/0148213 A1, intitulé « *Planning, navigation and simulation systems and methods for minimally invasive therapy* ». La méthode décrite dans cette demande de brevet détermine des trajectoires en utilisant un algorithme classique de traitement d'images dans lequel les images sont segmentées afin de pouvoir minimiser des contraintes afférentes à la trajectoire. Par exemple, lors d'une opération du cerveau, la trajectoire est déterminée par une optimisation de plusieurs paramètres, tels que la minimisation du nombre de fibres impactées, la distance entre une limite d'un sillon du cortex et la cible, le volume de matière blanche et/ou grise déplacé par la trajectoire.

Toutefois, l'inconvénient majeur des techniques de l'art antérieur est qu'elles se basent généralement sur une minimisation de contraintes sélectionnées par un opérateur afin de créer un modèle théorique, souvent incomplet et imparfait. En outre, elles nécessitent une segmentation systématique des images afin de pouvoir calculer au mieux les différentes trajectoires possibles. Cette segmentation s'avère imprécise et incomplète dans certains cas, ce qui peut induire des erreurs sur la trajectoire utilisée par l'instrument médical.

Par ailleurs, ces techniques ne permettent pas de tenir compte d'une éventuelle déformation de l'instrument médical, tel qu'une aiguille, lors de l'insertion de son extrémité dans le corps du patient.

Enfin, un opérateur expérimenté intervient également régulièrement afin de sélectionner dans les images les régions à éviter, telles que les vaisseaux sanguins, et les régions dans lesquelles l'instrument médical doit passer, afin de déterminer la trajectoire optimale de l'instrument médical.

Les interventions de l'opérateur s'avèrent fastidieuses et contraignantes car elles nécessitent une attention importante et de l'expérience de la part de l'opérateur dans le type de d'intervention.

Aucun des systèmes actuels ne permet de répondre simultanément à tous les besoins requis, à savoir de proposer une technique améliorée de planification automatique d'une intervention médicale visant à atteindre une cible dans une anatomie d'intérêt d'un patient qui soit indépendante d'un opérateur, tout en permettant d'obtenir une planification plus précise et plus fiable.

### EXPOSÉ DE L'INVENTION

La présente invention vise à remédier à tout ou partie des inconvénients de l'état de la technique cités ci-dessus.

À cet effet, l'invention vise un procédé de planification automatique d'une trajectoire à suivre au cours d'une intervention médicale par un instrument médical visant une anatomie d'intérêt d'un patient, ledit procédé de planification automatique comprenant des étapes de :
- acquisition d'au moins une image médicale de l'anatomie d'intérêt ;
- détermination d'un point cible sur l'image préalablement acquise ;
- génération d'un jeu de paramètres de planification de la trajectoire à partir de l'image de l'anatomie d'intérêt et du point cible préalablement déterminé, le jeu de paramètres de planification comprenant des coordonnées d'un point d'entrée sur l'image médicale.

Un tel procédé, utilisé en amont d'une intervention médicale, permet de fournir un jeu de paramètres guidant un médecin ou un chirurgien lors de la manipulation de l'instrument médical qui peut être une aiguille, une sonde, une électrode ou tout autre instrument médical susceptible d'être inséré dans le corps du patient, en utilisant un repère lié au patient. Ce repère est généralement tridimensionnel afin de guider l'instrument médical dans l'espace.

Le but de l'intervention médicale est d'atteindre une zone anatomique cible du corps du patient, afin par exemple d'ablater une tumeur dans un organe, de réaliser une biopsie, d'effectuer une vertébroplastie ou une cimentoplastie, ou de stimuler une zone anatomique. La zone anatomique cible se situe à l'intérieur ou à la surface d'une anatomie d'intérêt du patient. Une telle anatomie d'intérêt est par exemple un poumon, un rein, le foie, un tibia, un genou, une vertèbre ou le cerveau.

L'image médicale utilisée pour la planification a été réalisée par exemple par tomodensitométrie, par imagerie par résonance magnétique, par ultrasons, par tomographie par émission de positons ou par toute autre méthode d'imagerie médicale.

Selon l'invention, le procédé comprend également une phase d'entrainement d'un réseau de neurones sur un ensemble d'images médicales dites d'entraînement, chaque image d'entraînement comprenant une anatomie d'intérêt similaire à l'anatomie d'intérêt du patient, chaque image médicale d'entraînement étant associée à des coordonnées d'un point cible et d'au moins un point d'entrée préalablement déterminés, la génération du jeu de paramètres étant effectuée en mettant en oeuvre le réseau de neurones entraîné, le réseau de neurones entraîné générant dans un premier temps une probabilité d'être un point d'entrée pour chaque pixel ou voxel de l'image médicale acquise respectivement en 2D ou en 3D, les coordonnées du point d'entrée correspondant aux coordonnées du pixel ou du voxel ayant la plus grande probabilité.

Ainsi, le procédé de planification peut être utilisé par n'importe quel opérateur qui a juste à sélectionner un point cible sur l'image médicale.

Il convient de souligner que le procédé de planification est basé sur un apprentissage automatique d'images médicales similaires, associées chacune à un point d'entrée et à un point cible.

On entend par image médicale similaire, une image obtenue par une méthode d'imagerie identique ou équivalente et comprenant la même anatomie d'intérêt dans l'image médicale réalisée sur un individu quelconque. Il convient de souligner que le type d'intervention médicale, le type d'instrument médical ou l'anatomie d'intérêt visée peuvent être distincts sans préjudice sur la précision des paramètres de planification obtenus. L'apprentissage permet en effet d'analyser une nouvelle image afin de déterminer une trajectoire optimale à destination du point cible choisi par l'opérateur sur l'image médicale de l'anatomie d'intérêt du patient.

Il convient de souligner que les images médicales d'entraînement sont généralement associées à des points d'entrée effectivement utilisés lors de l'intervention médicale subie par les individus et des points cibles effectivement atteints par l'instrument suite à son insertion. Afin de compléter l'ensemble d'images médicales d'entraînement, des images médicales associés à des points d'entrées supposés, choisis par un opérateur, peuvent être ajoutées à l'ensemble.

En outre, le procédé de planification automatique se base avantageusement sur l'apprentissage d'images médicales non segmentées, c'est-à-dire dont tout ou partie de l'image est caractérisée selon le type de tissus, d'organes ou de vaisseaux présents dans la partie de l'image. Le traitement des images par le procédé de planification est ainsi plus rapide.

L'ensemble d'images médicales d'entraînement est généralement compris dans une base de données ou dans une banque d'images médicales.

Le procédé de planification automatique fournit généralement des paramètres de planification pour au moins une trajectoire possible. Lorsque le procédé de planification automatique fournit les paramètres de planification pour plusieurs trajectoires possibles, l'opérateur sélectionne généralement manuellement la trajectoire qui lui semble la meilleure. Il convient de souligner qu'une trajectoire est généralement considérée comme étant la meilleure lorsqu'elle répond à un certain nombre de critères spécifiques à l'intervention médicale, tels que l'angle d'incidence par rapport à une interface tissulaire (par exemple : la peau, la capsule du foie...), la proximité d'un vaisseau sanguin, d'organe ou de structure osseuse sur la trajectoire, etc.

Il convient de souligner que le procédé de planification automatique est mis en oeuvre avant tout geste médical, chirurgical ou thérapeutique.

Dans des modes de mise en oeuvre particuliers de l'invention, la méthode d'apprentissage automatique détermine les coordonnées du point d'entrée à partir de l'image médicale acquise et du point cible préalablement déterminé dans l'image médicale acquise.

Dans des modes de mise en oeuvre particuliers de l'invention, l'ensemble d'images médicales similaires comprend une pluralité d'images identiques, chaque image identique étant associée à un point d'entrée distinct.

Ainsi, l'apprentissage est amélioré car l'ensemble d'images médicales comprend des variantes de trajectoires possibles pour l'instrument médical.

Avantageusement, l'ensemble d'images médicales similaires comprend une pluralité d'images identiques, chaque image identique étant associée à un point d'entrée distinct choisi par un opérateur distinct.

Ainsi, les paramètres de planification obtenus sont plus précis car moins sensibles aux choix d'un opérateur particulier. Il convient de souligner que la précision des paramètres de planification obtenus est fonction du nombre d'opérateurs mis à contribution pour analyser la même image médicale lors de la phase d'apprentissage.

Préférentiellement, l'ensemble d'images médicales similaires comprend au moins trois images identiques, chaque image identique étant associée à un point d'entrée distinct par un opérateur distinct.

Ainsi, au moins trois opérateurs sont mis à contribution lors de la génération de la base de données comprenant l'ensemble d'images médicales utilisées lors de la phase d'apprentissage.

Dans des modes de mise en oeuvre particuliers de l'invention, une information relative à l'anatomie d'intérêt est associée à chaque image médicale de l'ensemble d'images médicales, l'information comprenant un type d'anatomie d'intérêt ou de tumeur présente dans l'anatomie d'intérêt, la méthode d'apprentissage automatique étant entraînée sur une partie de l'ensemble d'images médicales restreinte aux images associées au même type d'anatomie ou de tumeur.

Dans des modes de mise en oeuvre particuliers de l'invention, le procédé de planification automatique comprend également une étape d'attribution d'un score à une trajectoire définie entre le point d'entrée du jeu de paramètres de planification et le point cible préalablement déterminé sur l'image acquise.

Ainsi, l'opérateur est aidé dans son choix de trajectoire parmi les trajectoires possibles fournies par le procédé de planification automatique. Le score est généralement attribué selon des critères spécifiques à l'intervention médicale.

La trajectoire définie entre le point d'entrée du jeu de paramètres de planification et le point cible préalablement déterminé sur l'image acquise est généralement rectiligne. Toutefois, il peut être envisagée que la trajectoire soit curviligne, par exemple sensiblement selon un arc de cercle avec un rayon de courbure maximum afin de tenir compte de la rigidité de l'instrument médical. Généralement, une trajectoire curviligne est soit concave soit convexe. En d'autres termes, la dérivée d'une trajectoire curviligne est généralement de signe constant, négatif ou positif, entre le point d'entrée et le point cible.

Préférentiellement, l'attribution du score de la trajectoire est fonction d'au moins l'un des critères suivants :
- la proximité d'un vaisseau sanguin ;
- la proximité d'un organe ;
- la proximité d'une structure osseuse ;
- l'angle d'incidence par rapport à une interface tissulaire ;
- la longueur de la trajectoire ;
- la fragilité d'un tissu traversé par la trajectoire.

Dans des modes de mise en oeuvre particuliers de l'invention, l'attribution du score pour la trajectoire prend en compte une probabilité que l'instrument médical se déforme au contact d'une interface tissulaire.

Cette déformation intervient généralement lorsque l'instrument médical comporte une partie souple, c'est-à-dire susceptible de se déformer au contact d'une interface tissulaire, comme lors de l'insertion de l'insertion médical à travers la peau du patient.

Dans des modes de mise en oeuvre particuliers de l'invention, l'attribution du score pour la trajectoire prend en compte un taux de récidive ou un temps de récupération associé(s) à une trajectoire similaire à la trajectoire planifiée.

Ainsi, le score attribué à la trajectoire est impacté négativement si la trajectoire planifiée entraîne un taux de récidive ou un temps de récupération trop important pour le patient.

Dans des modes de mise en oeuvre particuliers de l'invention, le procédé de planification automatique comprend également une étape de comparaison du score attribué à la trajectoire avec un score seuil, la trajectoire étant validée lorsque le score de la trajectoire est supérieur ou égal au score seuil.

Dans des modes de mise en oeuvre particuliers de l'invention, le procédé de planification automatique comprend également une étape de modification du point d'entrée lorsque le score attribué à la trajectoire est inférieur au score seuil.

Dans des modes de mise en oeuvre particuliers de l'invention, l'image médicale acquise est bidimensionnelle ou tridimensionnelle.

Dans des modes de mise en oeuvre particuliers de l'invention, l'image médicale est acquise par résonance magnétique, par ultrasons, par tomodensitométrie ou par tomographie par émission de positions.

L'invention concerne également un dispositif de guidage d'un instrument médical, comprenant des moyens de guidage d'un instrument médical selon le jeu de paramètres de planification obtenus par le procédé de planification automatique selon l'un quelconque des modes de mise en oeuvre précédents.

Le dispositif de guidage peut être robotisé, un système de navigation associé ou non à un dispositif robotisé, un dispositif de réalité augmentée, un guide spécifique au patient ou un modèle tridimensionnel de l'anatomie du patient.

Il convient de souligner que le dispositif de guidage de l'instrument médical permet d'accompagner un praticien effectuant l'intervention médicale.

### BRÈVE DESCRIPTION DES FIGURES

D'autres avantages, buts et caractéristiques particulières de la présente invention ressortiront de la description non limitative qui suit d'au moins un mode de réalisation particulier des dispositifs et procédés objets de la présente invention, en regard des dessins annexés, dans lesquels :
- La figure 1 est une vue schématique d'une intervention médicale au cours de laquelle un instrument médical est guidé selon un jeu de paramètres établi par un procédé de planification automatique selon l'invention ;
- La figure 2 est un schéma synoptique d'un procédé de planification automatique selon un mode particulier de mise en oeuvre de l'invention ;
- La figure 3 est un exemple d'image médicale acquise au cours de la première étape du procédé de planification de la figure 2 ;
- La figure 4 est un exemple d'image médicale utilisée lors de l'entraînement du réseau de neurones mis en oeuvre par le procédé de la figure 2 ;
- La figure 5 est une vue schématique d'une phase d'entrainement du réseau de neurones mis en oeuvre par le procédé de la figure 2 ;
- La figure 6 est une vue schématique d'un déroulement du réseau de neurones mis en oeuvre par le procédé de la figure 2, et entraîné selon la phase d'entrainement de la figure 5 ;
- La figure 7 est une vue schématique d'un déroulement du réseau de neurones mis en oeuvre par le procédé de la figure 2, et entraîné selon une phase d'entrainement selon l'invention ;
- La figure 8 représente deux images médicales d'un même patient, l'une avec un instrument médical inséré ou l'autre correspondant à la même vue sans l'instrument médical, utilisées lors d'un apprentissage d'un réseau de neurones configurés pour définir une trajectoire curviligne d'un instrument médical.

### DESCRIPTION DETAILLEE DE L'INVENTION

La présente description est donnée à titre non limitatif, chaque caractéristique d'un mode de réalisation pouvant être combinée à toute autre caractéristique de tout autre mode de réalisation de manière avantageuse.

On note, dès à présent, que les figures ne sont pas à l'échelle.

### Exemple d'un mode de mise de réalisation particulier

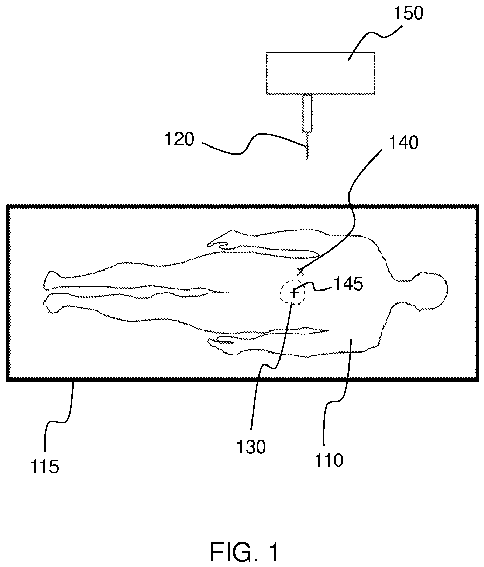

La figure 1 est une vue schématique d'une intervention médicale au cours de laquelle un patient 110 allongé sur une table 115 est soigné à l'aide d'un instrument médical 120. Dans le présent exemple non limitatif de l'invention, l'intervention médicale correspond à l'ablation d'une tumeur dans une anatomie d'intérêt 130 qui est ici le foie du patient 110 par l'intermédiaire de l'instrument médical 120 qui est dans le cas présent une aiguille semi-rigide. L'intervention médicale est ici une procédure percutanée au cours de laquelle le corps du patient 110 n'est pas ouvert. En outre, l'intervention médicale peut être réalisée selon différents paramètres de traitement. De tels paramètres de traitement sont par exemple une durée et une puissance du traitement de l'ablation, un voltage appliqué en cas d'un traitement par électroporation ou encore une fréquence appliquée dans le cas d'un traitement par radiofréquence. Il convient de souligner que le présent exemple est donné à titre illustratif et que l'homme du métier peut mettre en oeuvre l'invention décrite ci-après pour tout type d'intervention médicale mettant en oeuvre un instrument médical quelconque visant une anatomie d'intérêt du patient.

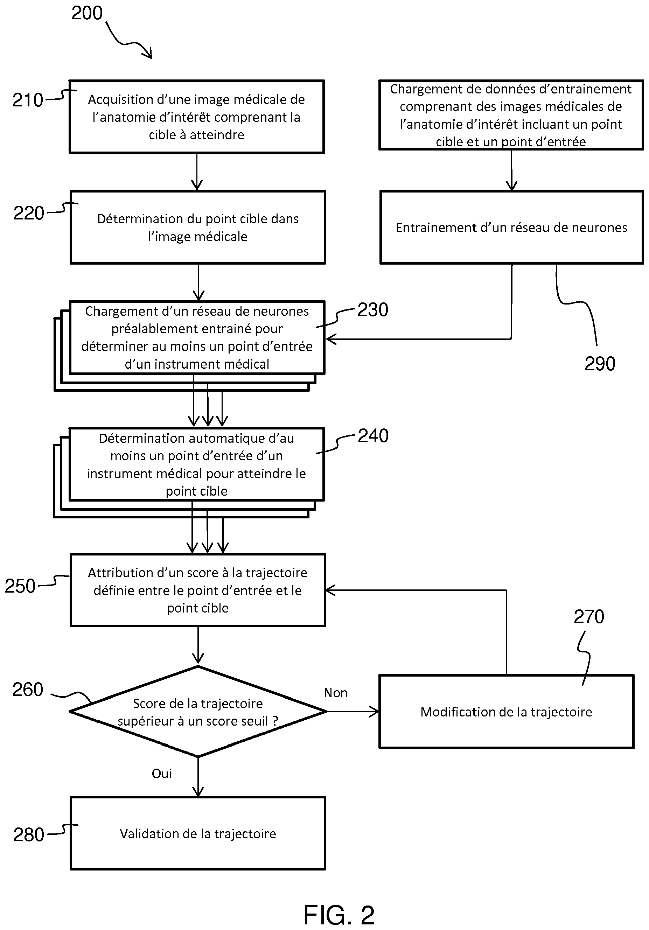

L'instrument médical 120 est dans le présent exemple avantageusement guidé par un dispositif 150 selon une trajectoire rectiligne grâce à l'élaboration préalable d'un jeu de paramètres de planification comprenant des coordonnées d'un point 140 d'entrée au niveau de la peau du patient 110, voire un angle à suivre dans un repère tridimensionnel lié au patient 110 pour viser un point cible 145 préalablement déterminé. Le jeu de paramètres de planification est établi par l'intermédiaire d'un procédé 200 de planification automatique selon l'invention, tel qu'illustré en figure 2 sous la forme d'un schéma synoptique.

Le procédé 200 de planification automatique de la trajectoire à suivre par l'instrument médical 120 au cours de l'intervention médicale comprend une première étape 210 d'acquisition d'au moins une image médicale de l'anatomie d'intérêt 130 du patient 110.

L'image médicale est généralement prise en amont de l'intervention médicale avec un équipement dédié à l'imagerie médicale, tel qu'un appareil d'imagerie par résonance magnétique (IRM), un tomodensitomètre, un scanner spectral ou un échographe.

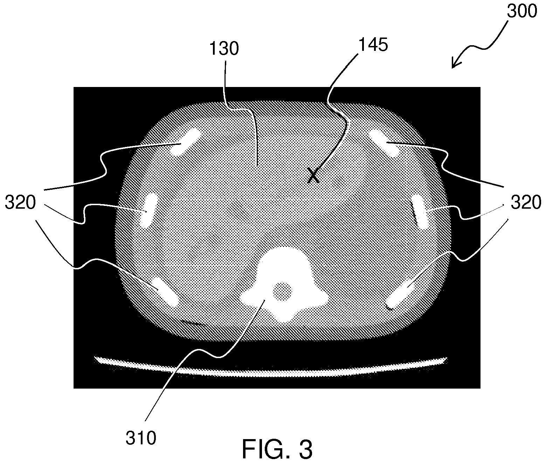

Un exemple d'image médicale 300 obtenue par tomodensitométrie montrant un modèle, couramment appelé par le terme anglais « *phantom* », correspondant à l'anatomie d'intérêt 130 du patient 110 est présenté en figure 3. L'image médicale 300 correspond à une vue en coupe du patient 110 selon un plan sensiblement perpendiculaire à l'axe de la colonne vertébrale du patient 110. Outre l'anatomie d'intérêt 130, sont notamment visibles dans l'image médicale 300 une vertèbre 310 de la colonne vertébrale et six côtes 320.

Dans l'image médicale 300 préalablement acquise, le point cible 145 est déterminé au cours d'une deuxième étape 220 du procédé 200 de planification automatique soit manuellement par un opérateur, soit automatiquement par une analyse d'images.

Le point cible 145 est associé à des coordonnées dans l'image médicale 300. Ces coordonnées sont bidimensionnelles ou tridimensionnelles suivant le type d'image médicale acquise. Dans le cas d'une image médicale 300 bidimensionnelle, le point cible 145 correspond sensiblement à un pixel de l'image. Dans le cas d'une image médicale 300 tridimensionnelle, le point cible 145 correspond sensiblement à un voxel de l'image.

Afin de déterminer les coordonnées d'un point d'entrée d'un jeu de paramètres de planification de la trajectoire à suivre par l'instrument médical 120 à partir de l'image médicale 300 et du point cible 145, un algorithme d'apprentissage automatique, ici de type réseau de neurones, est chargé au cours d'une troisième étape 230 du procédé 200 de planification automatique.

Le réseau de neurones a été préalablement entraîné lors d'une phase 290 d'apprentissage sur un ensemble d'images médicales d'entraînement comprenant chacune une anatomie d'intérêt similaire à l'anatomie d'intérêt 130. Les images médicales d'entraînement ont généralement été acquises sur une cohorte d'individus, chaque image médicale d'entraînement étant associée à des coordonnées d'un point cible et d'un point d'entrée préalablement déterminés généralement par au moins un opérateur.

Avantageusement, l'ensemble d'images médicales d'entraînement comprend plusieurs fois la même image médicale mais associée avec des points d'entrées distincts déterminés généralement par au moins trois opérateurs.

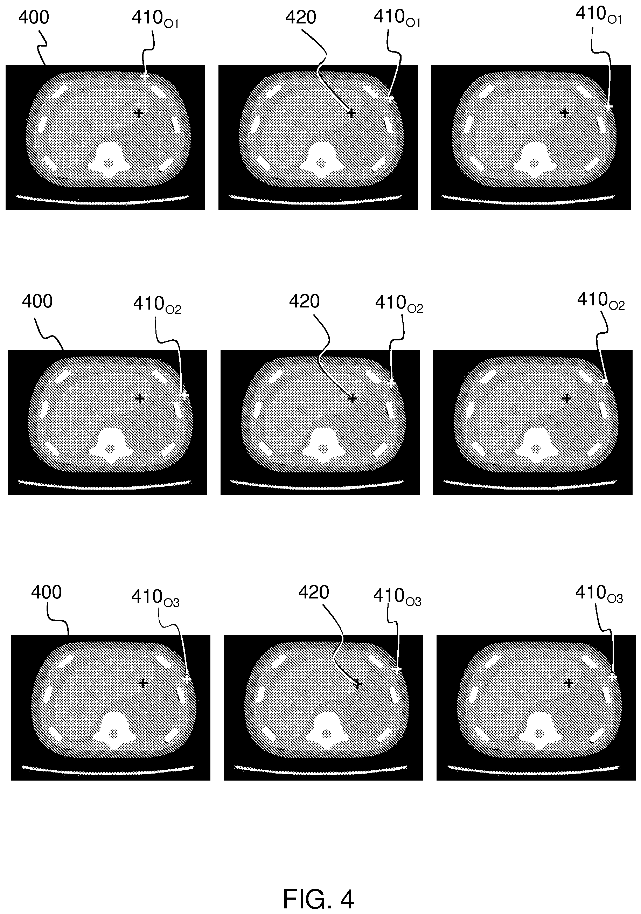

La figure 4 montre un exemple de la même image médicale 400 comprenant à chaque fois le même point cible 420. Cette image médicale 400 comprise neuf fois dans l'ensemble d'images médicales d'entraînement, a été traitée par trois opérateurs distincts, O1, O2 et O3, qui chacun a fourni trois points d'entrée, respectivement 410_{O1}, 410_{O2} et 410_{O3}.

L'entraînement du réseau de neurones peut être avantageusement restreint aux images associées à une information donnée, telle que le type d'anatomie d'intérêt ou de tumeur présente dans l'anatomie d'intérêt, afin d'augmenter la consistance en diminuant la variabilité des jeux de paramètres de planification que le réseau de neurones peut obtenir.

Il convient de souligner qu'il peut exister des limitations matérielles à l'entraînement d'un réseau de neurones, notamment lorsque l'ensemble d'images médicales d'entraînement comprend des images tridimensionnelles de l'anatomie d'intérêt. Afin de surmonter ces limitations matérielles, il est possible de réduire la résolution de chaque image médicale mais avec le risque de réduction de la précision des paramètres obtenus par le réseau de neurones. Il est également possible de restreindre l'entrainement aux trajectoires parallèles à un plan prédéterminé, tel qu'un plan perpendiculaire à l'axe de la colonne vertébral du patient. Une autre solution pour surmonter les limitations matérielles, peut consister à utiliser des puces couramment appelées par le terme anglais « *tensor processor units* », qui sont dédiées à l'apprentissage automatique, connu sous le terme anglais « *machine learning* »*.*

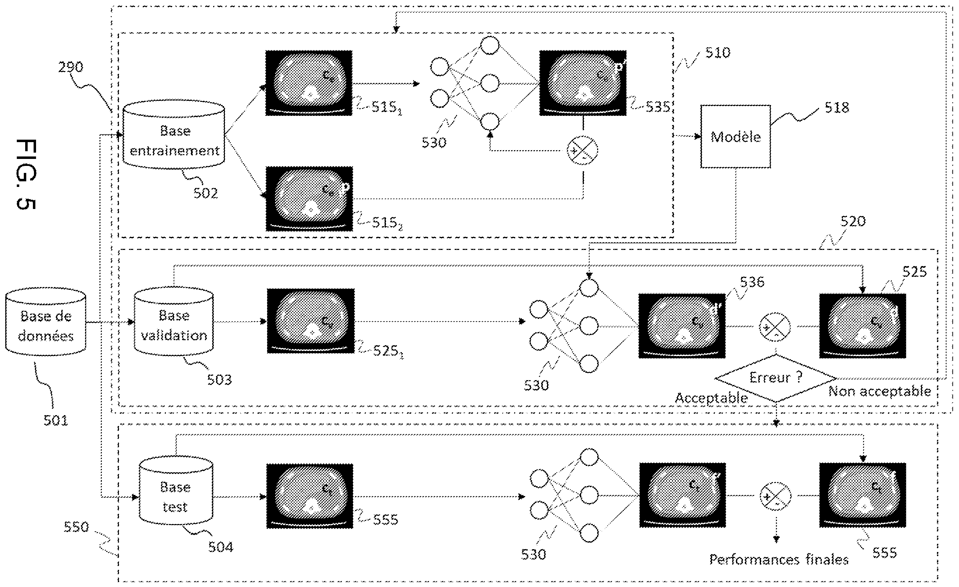

La phase 290 d'entraînement du réseau de neurones, tel qu'illustrée plus en détails en figure 5, comprend généralement deux étapes principales 510, 520, pouvant être répétées, et requiert une base de données 501 comprenant un ensemble d'images médicales où chaque image est associée à un point d'entrée et à un point cible. Eventuellement, une information sur les propriétés de l'instrument utilisé pour réaliser l'intervention, telle que la longueur de l'instrument ou le coefficient de rigidité de l'instrument, est également associée à chaque image médicale de la base de données 501. Après la phase 290 d'entraînement, une éventuelle phase de test 550 peut être mise en œuvre.

La base de données 501 d'image médicales est partitionnée en trois bases de données 502, 503, 504 comprenant des images médicales distinctes. Les trois bases de données 502, 503, 504 sont appelées respectivement base d'entraînement, base de validation et base de test.

Dans le présent exemple non limitatif de l'invention, 60 à 98 % des images médicales de la base de données 501 sont regroupées dans la base d'entraînement 502, 1 à 20 % dans la base de validation 503 et 1 à 20 % dans la base de test 504. Les pourcentages, fonctions généralement du nombre d'images de la base de données 501, sont donnés ici à titre indicatif.

Lors de la première étape 510 de la phase d'entraînement, des images médicales 515 de la base d'entraînement 502 sont utilisées pour déterminer un poids W et un biais b pour chaque neurone du réseau de neurones 530 utilisé pour obtenir les coordonnées du point d'entrée du jeu de paramètres de planification de la trajectoire.

Pour déterminer le poids W et le biais b de chaque neurone, chaque image médicale 515 de la base d'entraînement 502 est proposée au réseau de neurones 530 selon deux variantes, une première 515₁ comprenant uniquement le point cible cₑ, et une deuxième 515₂ comprenant à la fois le point cible cₑ et le point d'entrée p prédéterminé. A partir de la première variante de l'image médicale 515₁, le réseau de neurones 530 effectue alors une prédiction 535 sur la position du point d'entrée p'. Les coordonnées du point d'entrée prédit p' sont comparées aux coordonnées de la position du point d'entrée prédéterminé p, associé à la deuxième variante de l'image médicale 515₂. L'erreur entre les coordonnées du point d'entrée prédit p' et du point d'entrée prédéterminé p est ensuite utilisée pour ajuster les paramètres W et b de chaque neurone du réseau de neurones 530. Un modèle 518 est obtenu à l'issue de la première étape 510 de la phase d'entraînement.

Lors de la seconde étape 520 de la phase d'entraînement, les images médicales 525 de la base de validation 503, avantageusement distinctes des images médicales 515, sont utilisées pour valider le poids W et le biais b de chaque neurone du réseau de neurones 530.

Au cours de cette seconde étape 520 de la phase 290 d'entraînement, une variante 525₁ de chaque image médicale comprenant uniquement la position d'un point cible cᵥ est proposé au réseau de neurones 530. Le réseau de neurones 530 effectue alors une prédiction 536 sur la position du point d'entrée d'. Les coordonnées du point d'entrée prédit d' sont comparées aux coordonnées de la position du point d'entrée prédéterminé d, associé à l'image médicale 525 utilisée pour la validation. L'erreur entre les coordonnées du point d'entrée prédit d' et du point d'entrée prédéterminé d est ensuite utilisée pour vérifier les paramètres W et b de chaque neurone du réseau de neurones 530 déterminés lors de la première étape 510.

Dans le cas où l'erreur de prédiction du réseau de neurones serait trop importante à l'issue de cette seconde étape 520, le réseau de neurones 530 est ré-entrainé selon les deux étapes 510 et 520 de la phase 290 d'entrainement décrite précédemment, en réutilisant les mêmes images médicales d'entrainement 515 et de validation 525.

Alternativement, au cours du réentrainement du réseau de neurones 530, la première étape 510 utilise tout ou partie des images de validation 525. La seconde étape 520 de réentrainement du réseau de neurones utilise autant d'images d'entraiment 515 que d'images de validation 525 utilisées pour la première étape 510 de réentrainement.

Il convient de souligner que le réseau de neurones 530 peut être réentraîné autant de fois que nécessaire pour réduire l'erreur de prédiction.

Lorsque les deux étapes 510, 520 de la phase 290 d'entrainement sont mises en oeuvre au moins une fois, les performances finales du réseau de neurones peuvent être testées au cours d'une éventuelle phase 550 de test avec les images médicales 555 de la base de test 504. Ces images médicales 555, avantageusement distinctes des images 515 et 525 permettent de vérifier que le réseau de neurones 530 tel que configuré avec les paramètres W et b pour chaque neurone permet de prédire avec une bonne précision les coordonnées d'un point d'entrée dans toutes les situations auxquelles le réseau de neurones 530 est susceptible d'être confronté. Une comparaison est ainsi effectuée entre les coordonnées du point d'entrée f' telles que prédites par le réseau de neurones 530 et le point d'entrée f prédéterminé dans l'image médicale 555 dite de test. Cette comparaison est identique à celle effectuée au cours de la seconde étape 520 de la phase d'entrainement. Toutefois, à la différence de l'étape 520, cette phase 550 de test n'aboutit pas à un nouveau cycle d'entrainement du réseau de neurones 530. Si les performances du réseau de neurones 530 ne sont pas bonnes à l'issue de l'étape 550, la phase 290 d'entraînement est alors recommencée avec un nouveau réseau de neurones non entrainé.

Il convient de souligner que les images 555 utilisées lors de la phase 550 de test sont généralement soigneusement sélectionnées afin de couvrir différentes positions du point cible et dans l'anatomie d'intérêt afin de tester au mieux les capacités de prédiction du réseau d'entraînement 530.

Dans une phase d'entrainement selon l'invention, le réseau de neurones est entraîné à fournir pour chaque pixel ou voxel d'une image médicale, une probabilité qu'il corresponde effectivement au point d'entrée. L'ensemble d'images médicales utilisées pour cet entraînement alternatif peut être identique à l'ensemble d'images médicales utilisées précédemment. Cependant, il peut être préférable pour cet entraînement alternatif d'utiliser des images médicales présentant plusieurs points d'entrée sur la même image. Avantageusement, les points d'entrée affichés sur la même image sont déterminés par au moins trois opérateurs distincts. L'entrainement alternatif du réseau de neurones se déroule selon trois étapes similaires à la phase d'entraînement décrite précédemment.

Le réseau de neurones préalablement entraîné permet de déterminer au cours de la quatrième étape 240 du procédé 200 de planification automatique au moins un jeu de paramètres de planification de la trajectoire à suivre par l'instrument médical 120 à partir de l'analyse.

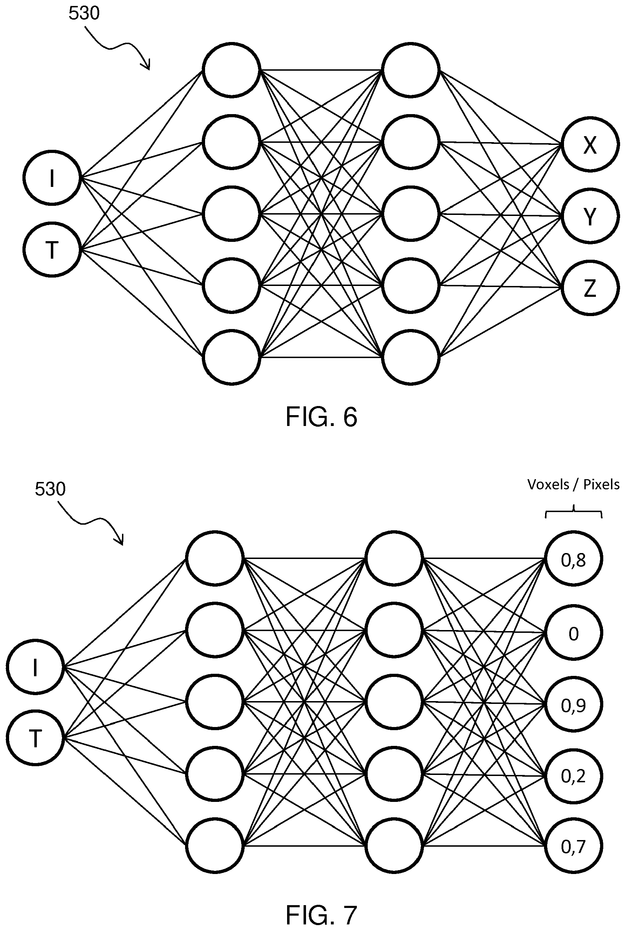

Dans le cas où le réseau de neurones est entraîné selon la phase 290 d'entrainement, le réseau de neurones 530 va fournir, à partir de l'image médicale I et des coordonnées du point cible T, des coordonnées tridimensionnelles (x,y,z) du point d'entrée dans l'image médicale acquise, comme illustré en figure 6.

Dans le cas où le réseau de neurones est entraîné selon la phase d'entraînement selon l'invention, le réseau de neurones 530 va fournir, à partir de l'image médicale I et des coordonnées du point cible T, une probabilité pour chaque pixel ou voxel de l'image médicale d'être le point d'entrée, comme illustré en figure 7. Le pixel ou le voxel ayant la probabilité la plus élevée est alors sélectionné comme étant le point d'entrée.

Le procédé 200 de planification automatique illustré en figure 2 comprend une cinquième étape 250 mise en oeuvre dès lors où une trajectoire est déterminée par le biais d'un jeu de paramètres de planification généré par le réseau de neurones. Au cours de cette cinquième étape 250, un score est attribué à la trajectoire définie par la droite reliant le point d'entrée et le point cible.

Par exemple, le score attribué à la trajectoire est compris entre 0 et 100, le score égal à 100 correspondant au score d'une trajectoire idéale.

Dans des variantes de ce mode de réalisation particulier de l'invention, la trajectoire est curviligne, obtenue par exemple en calculant la trajectoire la plus probable sur l'image médicale acquise, préalablement segmentée, ou par un réseau de neurones ayant préalablement appris les trajectoires suivies au cours d'interventions médicales antérieures par un instrument médical similaire ou identique notamment en termes de rigidité et de longueur. Le jeu de paramètres comprend alors des paramètres complémentaires permettant de définir la trajectoire prédite entre le point d'entrée et le point cible.

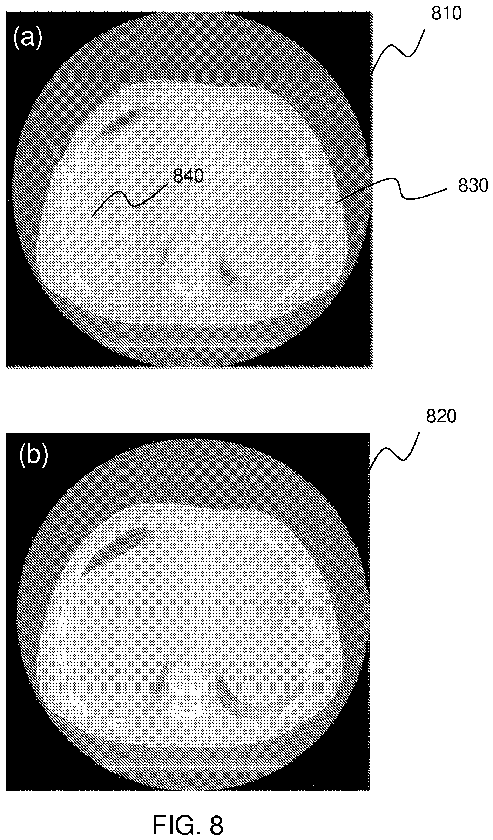

A titre d'illustration de ces variantes de mode de réalisation de l'invention, la figure 8 présente deux images médicales 810, 820 d'un patient 830 comprenant ou non un instrument médical 840. En effectuant une différence entre les deux images médicales 810 et 820, il est possible de déterminer la trajectoire effectivement prise par l'instrument médical 8840. Cette trajectoire peut également être déterminée en effectuant une reconnaissance de l'instrument médical 840 dans l'image médicale 810, par exemple en détectant de fortes variations d'intensité ou de contrastes au niveau des pixels/voxels de l'image médicale 810 afin de détourer l'instrument médical 840 dans l'image médicale 810.

Le score de la trajectoire est généralement déterminé à partir de critères pouvant être classés selon un ordre d'importance. Il convient de souligner que les exemples de critères décrits ci-après ne sont pas limitatifs et que d'autres critères spécifiques à une intervention médicale donnée peuvent être utilisés pour déterminer le score de la trajectoire.

Le score de la trajectoire peut par exemple être calculé en fonction de la proximité de la trajectoire avec un vaisseau sanguin. En effet, lorsque la trajectoire de l'instrument médical est susceptible de traverser un vaisseau sanguin, il y a un risque qu'une hémorragie survienne. En conséquence, plus le nombre de vaisseaux sanguins présents sur la trajectoire est important, plus le score attribué à la trajectoire est faible.

Il convient de souligner que la taille d'un vaisseau sanguin peut être pris en compte dans cette évaluation du score. Par exemple, si un vaisseau sanguin de diamètre supérieur ou égal à 3 mm se trouve sur ou à proximité de la trajectoire calculée par le réseau de neurones, des points sont automatiquement retranchés du score, par exemple 50 points sur l'échelle de 0 à 100, car ces vaisseaux sanguins peuvent s'avérer vitaux pour le patient. Lorsqu'un vaisseau sanguin traversé s'avère être une veine cave, une veine porte ou l'aorte, le score est automatiquement égal à 0, ce qui peut être le cas notamment lors d'une ablation d'une tumeur dans un foie.

Le score de la trajectoire peut également être calculé en fonction de la proximité de la trajectoire avec un organe et/ou une structure osseuse.

En effet, pour certaines interventions, par exemple sur un tissu mou, aucune structure osseuse ne doit se trouver sur la trajectoire. Si c'est le cas, le score attribué à la trajectoire est nul.

Pour d'autres interventions, par exemple sur des structures osseuses telles qu'un genou ou une épaule, la traversée d'une structure osseuse n'impacte pas négativement le score attribué. Plus précisément, si la trajectoire traverse une structure osseuse prédéterminée, le score attribué peut être augmenté.

Au regard des organes, le score de la trajectoire est généralement diminué lorsqu'un organe à risque, tel qu'un poumon, un intestin ou un muscle, se trouve au moins à proximité de la trajectoire. C'est également le cas lorsqu'un nerf, une voie biliaire, un ligament, un tendon ou un organe voisin de l'anatomie d'intérêt se trouve au moins à proximité de la trajectoire.

Le score de la trajectoire peut également être calculé en fonction de l'angle d'incidence de la trajectoire avec une interface tissulaire au niveau du point d'entrée.

Par exemple, dans le cas de l'insertion d'une aiguille semi-rigide selon une trajectoire tangentielle à une interface tissulaire, telle que la peau ou la capsule du foie, il y un risque que l'aiguille se courbe et ne suive pas la trajectoire planifiée. Plus l'angle entre la trajectoire et l'interface tissulaire est petit, plus le score de la trajectoire est faible. Le critère peut se traduire par le fait que la trajectoire optimale correspond à un angle entre l'interface tissulaire et la trajectoire supérieur à 20°.

Le score de la trajectoire peut également être calculé en fonction de l'angle d'incidence de la trajectoire avec une structure osseuse.

Par exemple, dans le cas d'une intervention sur une structure osseuse, il y a un risque que l'instrument médical dérape sur l'os lorsqu'il est inséré de façon tangentielle à l'os. Le critère se traduit alors par le fait que plus l'angle entre la trajectoire et la structure osseuse est élevé, plus le score de la trajectoire est faible.

Le score de la trajectoire peut également être calculé en fonction de la longueur de la trajectoire afin de minimiser la longueur de la trajectoire et le risque inhérent de créer un dommage dans le corps du patient.

Le score de la trajectoire peut également être calculé en fonction de la fragilité d'un tissu traversé.

Par exemple, dans le cas particulier d'une intervention sur le cerveau d'un patient, le score de la trajectoire peut être réduit si la trajectoire planifiée traverse des tissus fragiles.

Dans le cas d'une insertion d'aiguille semi-rigide, le score peut également être calculé en fonction d'une probabilité de déformation de l'aiguille lors de l'insertion. Cette probabilité est calculée à l'aide d'informations sur le type d'aiguille utilisée, telle que la longueur, le coefficient de rigidité ou la forme du biseau de l'aiguille, combinées aux informations déterminées précédemment, à savoir le type de tissus traversé, l'angle d'incidence et/ou la longueur de la trajectoire.

Il convient de souligner que pour calculer le score de la trajectoire, l'image médicale acquise peut avoir été préalablement segmentée afin d'identifier les différents types d'éléments présents dans l'image acquise, tel qu'un tissu, un vaisseau sanguin, une structure osseuse, etc., et se trouvant sur ou à proximité de la trajectoire définie entre le point d'entrée prédit et le point cible prédéterminé. Cette segmentation de l'image acquise est utilisée uniquement lorsque la trajectoire de l'instrument médical est générée et non lors de la génération de la trajectoire par le réseau de neurones.

Le score de la trajectoire, obtenu à partir des critères propres à l'intervention médicale, peut être pondéré en fonction d'un taux de récidive et/ou avec un temps de récupération.

Concernant le taux de récidive, le score obtenu est réduit lorsque la trajectoire planifiée par le réseau de neurones est similaire à une trajectoire utilisée avec les mêmes paramètres de traitement lors de précédentes interventions médicales mettant en oeuvre le même instrument médical, et pour laquelle le taux de récidive des individus ayant subi ces interventions médicales est notable.

De même, concernant le temps de récupération, le score obtenu est réduit lorsque le temps de récupération constaté précédemment pour des individus ayant subi une intervention médicale avec une trajectoire similaire à la trajectoire planifiée est élevé, par exemple supérieur à trois jours.

Le score attribué à la trajectoire planifié est ensuite comparé à un score seuil au cours d'une sixième étape 260 du procédé 200 de planification automatique.

Par exemple sur une échelle de 0 à 100, la trajectoire planifiée ne peut être validée que si le score attribué est supérieur ou égal à 50. De préférence, la trajectoire planifiée est validée si son score est supérieur ou égal à 70.

Dans le cas où le score attribué à la trajectoire est inférieur au score seuil, l'opérateur a la possibilité de modifier manuellement le point d'entrée au cours d'une éventuelle septième étape 270 du procédé 200 de planification automatique. La modification est effectuée par exemple via une interface graphique jusqu'à ce que le score de la trajectoire modifiée soit supérieur au score seuil.

Alternativement, la trajectoire peut être modifiée automatiquement à l'aide d'un algorithme de gradient, d'un algorithme de graphe, ou tout autre algorithme d'optimisation (Momentum, Nesterov Momentum, AdaGrad, RMSProp, Adam, etc.).

Enfin, lorsque le score de la trajectoire fournie par le réseau de neurones, éventuellement modifiée, est supérieur ou égal au score seuil, la trajectoire est validée au cours d'une huitième étape 280 du procédé 200 de planification automatique.

La trajectoire validée peut ensuite être utilisée au cours de l'intervention médicale pour guider l'insertion de l'instrument médical 120 dans l'anatomie d'intérêt 130 du patient 110 avec une très bonne précision et avec le maximum de chances que l'intervention médicale se déroule au mieux.

Il convient de souligner que le repère utilisé pour le guidage correspond généralement à la table 115 sur laquelle est allongé le patient 110. Les coordonnées du point cible sont avantageusement transférées dans le repère de guidage dans lequel des points caractéristiques du patient 110 ont été préalablement calibrés. Cette opération de transfert et de calibrage du repère de guidage est courante.

Le dispositif de guidage 150 peut alors être utilisé pour guider l'instrument médical 120 en suivant le jeu de paramètres de planification de la trajectoire validée.

Le dispositif de guidage 150 peut être robotisé, un système de navigation associé ou non à un dispositif robotisé, un dispositif de réalité augmentée, un guide spécifique au patient 110 ou un modèle tridimensionnel de l'anatomie du patient 110.

Le dispositif de réalité augmentée peut être par exemple une paire de lunettes dans laquelle la trajectoire planifiée est projetée sur au moins un des verres de la paire de lunettes. Le dispositif de réalité augmentée peut également être un écran placé à proximité du patient 110, l'écran affichant la trajectoire planifiée. Le dispositif de réalité augmentée peut également comprendre un projecteur projetant la trajectoire planifiée sur le corps du patient 110 ou être un dispositif holographique.

Le dispositif de guidage peut comprendre des moyens de navigation optique, des moyens de navigation électromagnétique ou une centrale inertielle comportant des capteurs d'accélération et de rotation.

Le jeu de paramètres de planification de la trajectoire validée peut être utilisé pour la construction d'un guide spécifique au patient 110. Ce guide spécifique est généralement utilisé dans le cadre d'une intervention médicale de type chirurgie ouverte d'une structure osseuse. Il convient de souligner que le guide spécifique au patient est un dispositif médical personnalisé à usage unique, généralement imprimé en 3D. Le guide spécifique au patient permet de prévenir des imprécisions lors de la chirurgie dans le but de réaliser une intervention telle que planifiée. Le guide spécifique épouse généralement la forme de la structure osseuse correspondant à l'anatomie d'intérêt et permet de guider l'insertion d'un instrument médical selon l'orientation de la trajectoire planifiée.

Dans le cas de certaines interventions médicales, un modèle tridimensionnel de l'anatomie du patient 110, couramment appelé par le terme anglais « *phantom* », est construit pour s'entraîner en amont de l'intervention médicale. Le modèle tridimensionnel de l'anatomie du patient 110 peut alors comprendre avantageusement une indication du point d'entrée de l'instrument médical tel que défini dans le jeu des paramètres de planification de la trajectoire obtenue par le procédé 200 de planification automatique.

Les résultats obtenus par le procédé 200 de planification automatique peuvent également être utilisés pour présenter l'intervention médicale à des pairs, tels que des médecins, des chirurgiens ou des radiologues, voire au patient 110 devant subir l'intervention médicale. Ces résultats peuvent également être utilisés pour former des pairs à l'intervention médicale.

## Revendications

1. Procédé (200) de planification automatique d'une trajectoire à suivre au cours d'une intervention médicale par un instrument médical (120) visant une anatomie d'intérêt (130) d'un patient (110), ledit procédé de planification automatique comprenant des étapes de :
- acquisition (210) d'au moins une image médicale (300) de l'anatomie d'intérêt ;
- détermination (220) d'un point cible (145) manuellement par un opérateur sur l'image (300) préalablement acquise ;
- génération (240) d'un jeu de paramètres de planification de la trajectoire à partir de l'image médicale de l'anatomie d'intérêt et du point cible préalablement déterminé, le jeu de paramètres de planification comprenant des coordonnées d'un point (140) d'entrée sur l'image médicale (300) ;
**caractérisé en ce qu'**il comprend également une phase (290) d'entrainement d'un réseau de neurones sur un ensemble d'images médicales dites d'entraînement, chaque image médicale d'entraînement comprenant une anatomie d'intérêt similaire à l'anatomie d'intérêt (130) du patient (110), chaque image médicale d'entraînement étant associée à des coordonnées d'un point cible et d'au moins un point d'entrée préalablement déterminés, la génération du jeu de paramètres étant effectuée en mettant en oeuvre le réseau de neurones (530) entraîné, le réseau de neurones entraîné générant dans un premier temps une probabilité d'être un point d'entrée pour chaque pixel ou voxel de l'image médicale acquise respectivement en 2D ou en 3D, les coordonnées du point d'entrée correspondant aux coordonnées du pixel ou du voxel ayant la plus grande probabilité.

2. Procédé de planification automatique selon la revendication 1, dans lequel l'ensemble d'images médicales similaires comprend une pluralité d'images identiques, chaque image identique étant associée à un point d'entrée distinct.

3. Procédé de planification automatique selon la revendication 1, dans lequel l'ensemble d'images médicales similaires comprend une pluralité d'images identiques, chaque image identique étant associée à un point d'entrée distinct choisi par un opérateur distinct.

4. Procédé de planification automatique selon l'une quelconque des revendications 1 à 3, dans lequel une information relative à l'anatomie d'intérêt est associée à chaque image médicale de l'ensemble d'images médicales, l'information comprenant un type d'anatomie d'intérêt ou de tumeur présente dans l'anatomie d'intérêt, le réseau de neurones étant entraîné sur une partie de l'ensemble d'images médicales restreinte aux images associées au même type d'anatomie ou de tumeur.

5. Procédé de planification automatique selon l'une quelconque des revendications 1 à 4, comprenant également une étape d'attribution d'un score à une trajectoire définie entre le point d'entrée du jeu de paramètres de planification et le point cible préalablement déterminé sur l'image acquise.

6. Procédé de planification automatique selon la revendication 5, dans lequel l'image acquise est segmentée, l'attribution du score de la trajectoire est fonction d'au moins l'un des critères suivants :
- la proximité d'un vaisseau sanguin ;
- la proximité d'un organe ;
- la proximité d'une structure osseuse ;
- l'angle d'incidence par rapport à une interface tissulaire ;
- la longueur de la trajectoire ;
- la fragilité d'un tissu traversé par la trajectoire.

7. Procédé de planification automatique selon l'une quelconque des revendications 5 à 6, dans lequel l'attribution du score pour la trajectoire prend en compte une probabilité que l'instrument médical se déforme au contact d'une interface tissulaire.

8. Procédé de planification automatique selon l'une quelconque des revendications 5 à 7, dans lequel l'attribution du score pour la trajectoire prend en compte un taux de récidive associé à une trajectoire similaire à la trajectoire planifiée.

9. Procédé de planification automatique selon l'une quelconque des revendications 5 à 8, dans lequel l'attribution du score pour la trajectoire prend en compte un temps de récupération associé à une trajectoire similaire à la trajectoire planifiée

10. Procédé de planification automatique selon l'une quelconque des revendications 5 à 9, comprenant également une étape de comparaison du score attribué à la trajectoire avec un score seuil, la trajectoire étant validée lorsque le score de la trajectoire est supérieur ou égal au score seuil.

11. Procédé de planification automatique selon l'une quelconque des revendications 5 à 10, comprenant également une étape de comparaison du score attribué à la trajectoire avec un score seuil, et une étape de modification du point d'entrée lorsque le score attribué à la trajectoire est inférieur au score seuil.

## Patentansprüche

1. Verfahren (200) zur automatischen Planung eines während eines medizinischen Eingriffs von einem eine interessierende Anatomie (130) eines Patienten (110) anpeilenden medizinischen Instrument (120) zu verfolgenden Pfads, wobei das automatische Planungsverfahren Schritte aufweist der:
- Erfassung (210) mindestens eines medizinischen Bilds (300) der interessierenden Anatomie;
- manuelle Bestimmung (220) eines Zielpunkts (145) auf dem vorher erfassten Bild (300) durch einen Operateur;
- Erzeugung (240) eines Satzes von Planungsparametern des Pfads ausgehend vom medizinischen Bild der interessierenden Anatomie und vom vorher bestimmten Zielpunkt, wobei der Satz von Planungsparametern Koordinaten eines Eingangspunkts (140) auf dem medizinischen Bild (300) enthält;
**dadurch gekennzeichnet, dass** es auch eine Trainingsphase (290) eines Neuronennetzes an einer Gesamtheit medizinischer so genannter Trainingsbilder enthält, wobei jedes medizinische Trainingsbild eine interessierende Anatomie ähnlich der interessierenden Anatomie (130) des Patienten (110) enthält, wobei jedes medizinische Trainingsbild Koordinaten eines Zielpunkts und mindestens eines Eingangspunkts zugeordnet ist, die vorher bestimmt wurden, wobei die Erzeugung des Satzes von Parametern ausgeführt wird, indem das trainierte Neuronennetz (530) eingesetzt wird, wobei das trainierte Neuronennetz zuerst eine Wahrscheinlichkeit generiert, ein Eingangspunkt für jedes Pixel oder Voxel des in 2D bzw. 3D erfassten erfassten medizinischen Bilds zu sein, wobei die Koordinaten des Eingangspunkts den Koordinaten des Pixels oder des Voxels entsprechen, das die größte Wahrscheinlichkeit hat.

2. Automatisches Planungsverfahren nach Anspruch 1, wobei die Gesamtheit ähnlicher medizinischer Bilder eine Vielzahl gleicher Bilder enthält, wobei jedes gleiche Bild einem unterschiedlichen Eingangspunkt zugeordnet ist.

3. Automatisches Planungsverfahren nach Anspruch 1, wobei die Gesamtheit ähnlicher medizinischer Bilder eine Vielzahl gleicher Bilder enthält, wobei jedes gleiche Bild einem von einem unterschiedlichen Operateur ausgewählten unterschiedlichen Eingangspunkt zugeordnet ist.

4. Automatisches Planungsverfahren nach einem der Ansprüche 1 bis 3, wobei eine Information bezüglich der interessierenden Anatomie jedem medizinischen Bild der Gesamtheit medizinischer Bilder zugeordnet ist, wobei die Information einen Typ einer interessierenden Anatomie oder eines in der interessierenden Anatomie vorhandenen Tumors enthält, wobei das Neuronennetz an einem Teil der Gesamtheit medizinischer Bilder trainiert wird, der auf die Bilder beschränkt ist, die dem gleichen Typ von Anatomie oder Tumor zugeordnet sind.

5. Automatisches Planungsverfahren nach einem der Ansprüche 1 bis 4, das ebenfalls einen Schritt der Zuweisung eines Scores zu einem Pfad enthält, der zwischen dem Eingangspunkt des Satzes von Planungsparametern und dem vorher auf dem erfassten Bild bestimmten Zielpunkt definiert ist.

6. Automatisches Planungsverfahren nach Anspruch 5, wobei das erfasste Bild segmentiert wird, die Zuweisung des Scores des Pfads von mindestens einem der folgenden Kriterien abhängt:
- die Nähe eines Blutgefäßes;
- die Nähe eines Organs;
- die Nähe einer Knochenstruktur;
- der Einfallwinkel bezüglich einer Gewebeschnittstelle;
- die Länge des Pfads;
- die Schwäche eines vom Pfad durchquerten Gewebes.

7. Automatisches Planungsverfahren nach einem der Ansprüche 5 bis 6, wobei die Zuweisung des Scores für den Pfad eine Wahrscheinlichkeit berücksichtigt, dass das medizinische Instrument sich bei Kontakt mit einer Gewebeschnittstelle verformt.

8. Automatisches Planungsverfahren nach einem der Ansprüche 5 bis 7, wobei die Zuweisung des Scores für den Pfad eine einem Pfad ähnlich dem geplanten Pfad zugeordnete Rezidiverate berücksichtigt.

9. Automatisches Planungsverfahren nach einem der Ansprüche 5 bis 8, wobei die Zuweisung des Scores für den Pfad eine einem Pfad ähnlich dem geplanten Pfad zugeordnete Erholungszeit berücksichtigt.

10. Automatisches Planungsverfahren nach einem der Ansprüche 5 bis 9, das ebenfalls einen Schritt des Vergleichs des dem Pfad zugewiesenen Scores mit einem Schwellenscore enthält, wobei der Pfad validiert wird, wenn der Score des Pfads höher als der oder gleich dem Schwellenscore ist.

11. Automatisches Planungsverfahren nach einem der Ansprüche 5 bis 10, das ebenfalls einen Schritt des Vergleichs des dem Pfad zugewiesenen Scores mit einem Schwellenscore und einen Schritt der Änderung des Eingangspunkts enthält, wenn der dem Pfad zugewiesene Score niedriger ist als der Schwellenscore.

## Claims

1. Method (200) for automatically planning a trajectory to be followed during a medical intervention by a medical instrument (120) targeting an anatomy of interest (130) of a patient (110), said automatic planning method comprising the steps of:
- acquiring (210) at least one medical image (300) of the anatomy of interest;
- determining (220) a target point (145), manually by an operator, on the previously acquired image (300);
- generating (240) a set of trajectory planning parameters from the medical image of the anatomy of interest and from the previously determined target point, the set of planning parameters comprising coordinates of an entry point (140) on the medical image (300);
**characterized in that** it also comprises a phase (290) of training a neural network on a set of medical training images, each medical training image comprising an anatomy of interest similar to the anatomy of interest (130) of the patient (110), each medical training image being associated with coordinates of a target point and of at least one entry point that have been previously determined, the set of parameters being generated using the trained neural network (530), the trained neural network initially generating a probability of being an entry point for each pixel or voxel of the medical image acquired in 2D or 3D respectively, the coordinates of the entry point corresponding to the coordinates of the pixel or voxel having the greatest probability.

2. Automatic planning method according to Claim 1, in which the set of similar medical images comprises a plurality of identical images, each identical image being associated with a distinct entry point.

3. Automatic planning method according to Claim 1, in which the set of similar medical images comprises a plurality of identical images, each identical image being associated with a distinct entry point chosen by a distinct operator.

4. Automatic planning method according to any one of Claims 1 to 3, in which information relating to the anatomy of interest is associated with each medical image of the set of medical images, the information comprising a type of anatomy of interest or tumour present in the anatomy of interest, the neural network being trained on a number of the set of medical images restricted to the images associated with the same type of anatomy or tumour.

5. Automatic planning method according to any one of Claims 1 to 4, also comprising a step of allocating a score to a trajectory defined between the entry point of the set of planning parameters and the target point previously determined on the acquired image.

6. Automatic planning method according to Claim 5, in which the acquired image is segmented, the allocation of the trajectory score being a function of at least one of the following criteria:
- the proximity of a blood vessel;
- the proximity of an organ;
- the proximity of a bone structure;
- the angle of incidence with respect to a tissue interface;
- the length of the trajectory;
- the fragility of a tissue through which the trajectory passes.

7. Automatic planning method according to either of Claims 5 and 6, in which the allocation of the trajectory score takes into account a probability of the medical instrument deforming upon contact with a tissue interface.

8. Automatic planning method according to any one of Claims 5 to 7, in which the allocation of the trajectory score takes into account a recurrence rate associated with a trajectory similar to the planned trajectory.

9. Automatic planning method according to any one of Claims 5 to 8, in which the allocation of the trajectory score takes into account a recovery time associated with a trajectory similar to the planned trajectory.

10. Automatic planning method according to any one of Claims 5 to 9, also comprising a step in which the score allocated to the trajectory is compared with a threshold score, the trajectory being validated when the trajectory score is greater than or equal to the threshold score.

11. Automatic planning method according to any one of Claims 5 to 10, also comprising a step in which the score allocated to the trajectory is compared with a threshold score, and a step of modifying the entry point when the score allocated to the trajectory is below the threshold score.
